# FASCICULE DE BREVET EUROPEEN

(11) **EP 1 065 975 B1**
(45) Date de publication et mention de la délivrance du brevet: **20.12.2006**
(21) Numéro de dépôt: 99911869.8
(22) Date de dépôt: 02.04.1999
(51) Int. Cl.: A61B 6/03

(54) **PROCEDE D'ACQUISITION DE MESURES ET TOMODENSITOMETRE POUR METTRE EN OEUVRE LE PROCEDE**
MESSERFASSUNGSVERFAHREN SOWIE EIN RÖNTGENCOMPUTERTOMOGRAPH ZUR DURCHFÜRUNG DES VERFAHRENS
METHOD FOR ACQUIRING MEASUREMENTS AND SCANNER IMPLEMENTING SAID METHOD

(30) Priorité: 03.04.1998 FR 9804180
(43) Date de publication de la demande: 10.01.2001
(73) Titulaire: THALES ELECTRON DEVICES S.A., 78140 Vélizy (FR)
(72) Inventeur: MUNIER, Bernard, Thomson-CSF Prop. Int. Dépt. Bre, 94117 Arcueil Cédex (FR); ROZIERE, Guy, Thomson-CSF Prop. Int. Dépt. Breve., 94117 Arcueil Cédex (FR)
(74) Mandataire: Guérin, Michel
(86) Numéro de dépôt international: PCT/FR1999/000776
(87) Numéro de publication internationale: WO 1999/051146

(56) Documents cités:
- EP-A- 0 230 155
- US-A- 5 212 737

## Description

L'invention concerne un procédé d'acquisition de mesures avec un tomodensitomètre à rayons X, et un tomodensitomètre pour mettre en oeuvre le procédé. Ces appareils utilisent généralement une seule source de rayons X et un détecteur à éléments multiples, opposé à la source. L'ensemble de la source et du détecteur peut tourner et ou se déplacer en translation par rapport au corps d'un patient qui est placé entre la source et le détecteur.

En général, le détecteur de rayons X comporte une multiplicité d'éléments de détection individuels, juxtaposés de manière à couvrir tout un arc de cercle éclairé par la source de rayons X. Il existe néanmoins des détecteurs qui ne sont pas courbes. Les détecteurs les plus connus sont les détecteurs à gaz et les détecteurs solides. Les signaux fournis par les éléments détecteurs sont recueillis périodiquement, numérisés, et transmis à un ordinateur au fur et à mesure de la rotation, et ou de la translation de l'appareil, pour être traités par calcul numérique afin de reconstruire une image interne d'une tranche du corps exposé aux rayons X.

Pour une plus grande résolution d'image, il est souhaitable d'avoir un grand nombre d'éléments de détection juxtaposés le long d'un arc de cercle, ou plus généralement d'une ligne située dans un plan sensiblement perpendiculaire à l'axe de rotation translation. D'autre part, le détecteur peut être constitué sous forme de plusieurs ensembles de détection juxtaposés dans une direction parallèle à l'axe de rotation translation. Dans ce cas, les éléments de détection individuels peuvent être agencés en plusieurs barrettes de deux ou plusieurs éléments. Ces éléments sont alignés dans une direction parallèle à l'axe de rotation, les barrettes étant juxtaposées le long de l'arc de cercle. Ceci permet notamment d'explorer simultanément deux ou plusieurs tranches très fines, juxtaposées, du corps examiné, puisque la source, en tournant, expose simultanément deux ou plusieurs séries adjacentes de détecteurs de petite dimension. Un tel appareil est par exemple décrit dans le document US-A- 5 592 523.

Le document EP. 0 230 155 décrit un appareil de radiographie ainsi que le procédé associé comme définit dans le préambule de la revendication 1.

A titre d'exemple, un tomodensitomètre peut utiliser comme éléments de détection jusqu'à plusieurs milliers de photodiodes, du type polarisé ou du type photovoltaïque, recouvertes individuellement d'un cristal scintillateur, et ce nombre pourrait encore augmenter jusqu'à plusieurs dizaines de milliers. L'exemple qu'on étudiera ici comportera 25000 éléments de détection environ. Il en résulte des difficultés pour recueillir les signaux électriques issus de ces nombreux éléments de détection. L'invention a pour but de proposer un mode de fonctionnement permettant de résoudre au mieux les contraintes techniques résultant de la présence de ce grand nombre d'éléments de détection.

Parmi ces contraintes, il y a :
- la nécessité de lire les signaux de tous les éléments de détection pendant une durée très brève, par exemple de l'ordre de 0,5 millisecondes. En effet un tomodensitomètre tourne en permanence à une vitesse, par exemple classique, de deux tours par seconde. Dans un exemple préféré, on veut pour chaque tour effectuer environ 1000 vues du corps. Une vue correspond à une durée donnée au cours de laquelle le corps est exposé au rayonnement X, d'une manière continue ou pulsée. L'image de la vue révélée sur le détecteur correspond au phénomène d'absorption radiologique qui se produit pendant la durée de la vue. On distingue l'image de la vue elle-même (relevée directement en projection par le détecteur), et l'image reconstruite d'une coupe du corps à laquelle cette vue participe dans les calculs de reconstruction. Pendant la durée d'une vue, on considère pour un traitement de l'image que le tomodensitomètre occupe une position fixe par rapport au corps. Bien entendu ce n'est pas vrai puisque le tomodensitomètre est en mouvement permanent. Une vue est néanmoins ainsi associée à une incidence, une position : le repérage de l'angle moyen sous lequel un axe principal de rayonnement X irradie le corps. Si la durée de mesure, la durée de vue, était plus grande la résolution finale de l'image ne serait pas acceptable : il faudrait alors soit prendre moins de vues, soit faire tourner le tomodensitomètre moins vite;
- la nécessité de lire des signaux sur une dynamique très grande, par exemple de l'ordre de 20 bits, car les parties sombres de l'image fournissent un signal extrêmement faible par rapport aux parties les plus claires, et ce signal faible doit cependant être mesuré avec une certaine résolution (quelques bits). Ainsi pour mesurer à la fois 1 000 000 photons X reçus en nominal, ou 4 photons X reçus au minimum, il faudrait une dynamique de mesure de 20 bits. Mais, si la dynamique globale de l'image représente ainsi 20 bits, la dynamique locale utile est moins exigeante. En effet, cette dynamique locale utile est égale au rapport signal sur bruit. Or, le rapport signal sur bruit vaut environ 1 000 au maximum. Il est en effet égal à la racine carrée du nombre de photons X reçus du fait du phénomène quantique d'absorption des rayons X dans le corps et de leur conversion dans un scintillateur. Ceci veut dire que sur les 20 bits mesurés, seuls 14 bits sont significatifs. La numérisation des mesures sur une telle dynamique implique néanmoins de retenir des circuits surdimensionnés en performance, et occupant trop de place dans un circuit intégré ;
- dans un détecteur solide, la nécessité de traiter des quantités de charge pouvant être assez élevées lorsque le détecteur reçoit des flux de rayonnement X importants. Des charges nominales de l'ordre de 100 picoCoulombs, pC, par photodiode sont en effet rencontrées. De telles charges nécessitent des surfaces importantes de stockage. Or ceci pose des problèmes de taille pour atteindre les capacités requises de stockage de ces charges, compte tenu des faibles tensions de fonctionnement de ces circuits intégrés. A titre d'exemple, les détecteurs peuvent être des diodes du type polarisé en inverse, un condensateur étant constitué aux bornes d'une diode du fait de sa polarisation inverse. Un préamplificateur peut être connecté successivement à chacune de ces diodes pour recharger le condensateur des charges que ce dernier a perdu sous l'action de la lumière reçue par la diode. La quantité de charges réinjectées par le préamplificateur forme le signal de mesure. De préférence, pour des raisons de linéarité, on utilisera des diodes non polarisées, de type photovoltaïque. Elles produisent un courant continu proportionnel à l'éclairement qu'elles reçoivent. La mesure du rayonnement X y est constituée par une liaison de cette diode photovoltaïque à un intégrateur, et par l'intégration pendant une petite durée de ce signal de courant. A chaque nouvelle durée l'intégrateur est préalablement remis à zéro. Dans ce dernier cas l'intégrateur est l'organe de stockage des charges à mesurer alors que c'est le condensateur constitué en inverse dans le cas précédent.

Pour permettre la réalisation d'un appareil respectant ces contraintes sans impliquer un coût de fabrication prohibitif, la présente invention propose un procédé d'obtention d'images tomographiques, utilisant de préférence au moins un réseau de détecteurs élémentaires, un élément de stockage de charge associé à chaque détecteur, et un circuit, un multiplexeur, associé au réseau d'éléments de stockage pour commander périodiquement la connexion des différents détecteurs à leurs éléments de stockage. Un convertisseur analogique-numérique transforme en aval en signaux numériques les signaux analogiques successifs fournis par le multiplexeur.

Le principe de l'invention consiste alors, au cours d'une vue, à morceler le temps d'intégration du signal afin de réduire la quantité de charges à mesurer. En effet, l'organe détecteur utilisé n'a plus besoin dans ces conditions d'être capable d'accumuler des charges en quantités importantes. Par ailleurs, le convertisseur analogique numérique n'a plus besoin d'une dynamique aussi importante. On montrera qu'on passe ainsi de 20 bits à 14 bits. Le fait de provoquer alors des échantillonnages et des quantifications multiples au cours d'une vue, conduit bien entendu, à la nécessité d'exécuter une sommation des résultats de quantification pour produire un signal correspondant à la vue, pour chaque détecteur.

Cependant, on aboutit quand même au résultat recherché qui est que :
- la capacité requise pour les éléments de stockage est réduite d'un facteur n (n étant un facteur d'accélération des mesures),
- la fréquence du convertisseur analogique numérique est augmentée du facteur n,
- la dynamique de ce convertisseur analogique numérique est, globalement, réduite dans un facteur compris entre n et racine de n.

L'invention a donc pour objet un procédé d'acquisition de mesures avec un tomodensitomètre comprenant les étapes suivantes
- on irradie un corps, pendant une vue donnée, avec un rayonnement X,
- on mesure pour cette vue, dans chaque détecteur d'un réseau de détecteurs, un signal analogique représentant l'effet de l'absorption du rayonnement X dans le corps à l'endroit de chacun de ces détecteurs,
- on échantillonne et on convertit chaque signal analogique de détecteur en un signal numérique de détecteur,
caractérisé en ce que
- on échantillonne et on convertit en numérique le signal analogique de chaque détecteur à n reprises au cours de la vue, et
- on additionne les n signaux convertis de chaque détecteur pour constituer le signal numérique de vue de chaque détecteur.

Elle a également pour objet un tomodensitomètre muni d'un dispositif d'acquisition de mesures comportant
- un tube à rayons X pour irradier avec un rayonnement X un corps pendant des vues successives,
- un réseau de détecteurs pour mesurer un signal analogique représentant l'effet de l'absorption du rayonnement X dans le corps à l'endroit de chacun de ces détecteurs pendant une vue,
- un convertisseur analogique numérique pour échantillonner et convertir chaque signal analogique de détecteur en un signal numérique de détecteur,
- un séquenceur pour piloter le réseau de détecteurs et le convertisseur au rythme des vues
caractérisé en ce qu'il comporte
- des moyens dans le séquenceur pour piloter le réseau de détecteurs et le convertisseur à un rythme n fois plus élevé que le rythme des vues, et
- un additionneur pour additionner les n signaux convertis de chaque détecteur pour constituer le signal numérique de vue de chaque détecteur.

Un autre problème à résoudre avec de tels tomodensitomètres est lié au nombre de leurs détecteurs, dont on a dit ci-dessus qu'il pouvait être très important. L'ensemble des circuits de commande de tous ces détecteurs constitue alors un système très volumineux à réaliser, même en mettant en oeuvre des techniques de miniaturisation les plus modernes. On ne passe pas facilement dans la réalité de la fabrication d'un détecteur avec 700 éléments de détection à un détecteur avec 25000 éléments de détection.

Pour résoudre cet autre problème, selon une autre caractéristique de l'invention, on constitue des groupements de détecteurs et on applique à tous les détecteurs de ces groupes des traitements communs. L'invention part en effet des principes suivants qu'elle a mis en évidence. Premièrement, dans une image en projection, l'évolution du contraste n'est jamais brutale, même si la dynamique de mesure sur toute l'image est, elle, grande. En effet, les organes du corps d'un patient soit s'interpénètrent soit sont vus en projection au travers d'autres organes. De ce fait, il existe toujours une zone de transition entre les images de ces organes. Cette zone de transition est relativement grande à l'échelle de la taille des détecteurs. Pour cette zone de transition, on peut alors considérer que le contraste n'évolue pas excessivement.

Deuxièmement, et à titre de complément, du fait de la rotation lente du tomodensitomètre, on peut considérer que le phénomène d'absorption mesuré dans un détecteur, au moment d'une vue, sera le même phénomène (avec une même dynamique) que le phénomène qui se produit dans un détecteur voisin à une vue suivante.

Autrement dit, dans l'invention, on a alors considéré qu'on pouvait déjà grouper les détecteurs d'une région. Ce groupement a pour effet de soumettre les détecteurs d'un groupe à un même mode de mesure. En pratique, ces considérations ont conduit à constituer des groupes de détecteurs pour lesquels la dynamique de mesure pourra être considérée comme comprise dans une même gamme. Selon l'invention, on attribue alors à chaque groupe de détecteurs une gamme de mesure. Ceci mène à la simplification des circuits électroniques de détection. La gamme est déterminée en veillant à ce que la plus grande mesure effectuée pour un détecteur d'un groupe soit comprise dans la plus petite gamme de mesure possible affectée à ce groupe. A titre de perfectionnement, la détermination est effectuée pendant une vue, et l'affectation est réalisée à la vue suivante.

L'invention sera mieux comprise à la lecture de la description qui suit et à l'examen des figures qui l'accompagnent. Celles-ci ne sont données qu'à titre indicatif et nullement limitatif de l'invention. Les figures montrent :
- Figure 1: la représentation schématique des moyens essentiels d'un tomodensitomètre;
- Figure 2: une représentation de la modification du circuit de détection d'un tomodensitomètre pour lui permettre de mettre en oeuvre le procédé de l'invention;
- Figures 3a à 3g: des diagrammes temporels de signaux mis en oeuvre respectivement dans l'état de la technique et dans l'invention, et correspondant au procédé;
- Figure 4 : un diagramme montrant différents modes d'utilisation du procédé de l'invention en fonction de la valeur des signaux détectés;
- Figure 5 : une représentation schématique de la réalisation d'un module de détection utilisé dans l'invention.

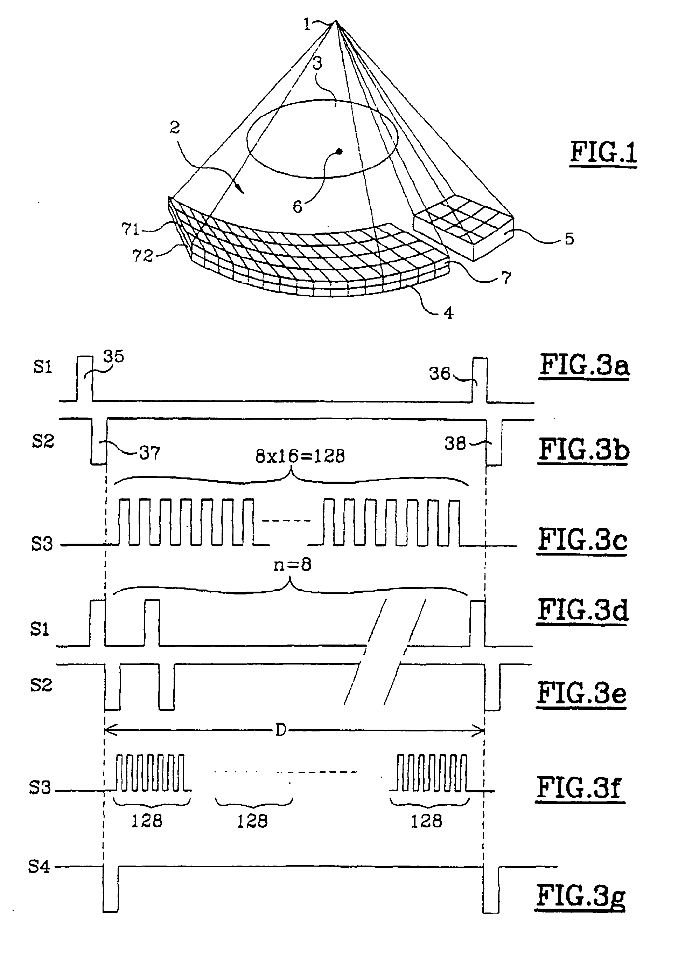

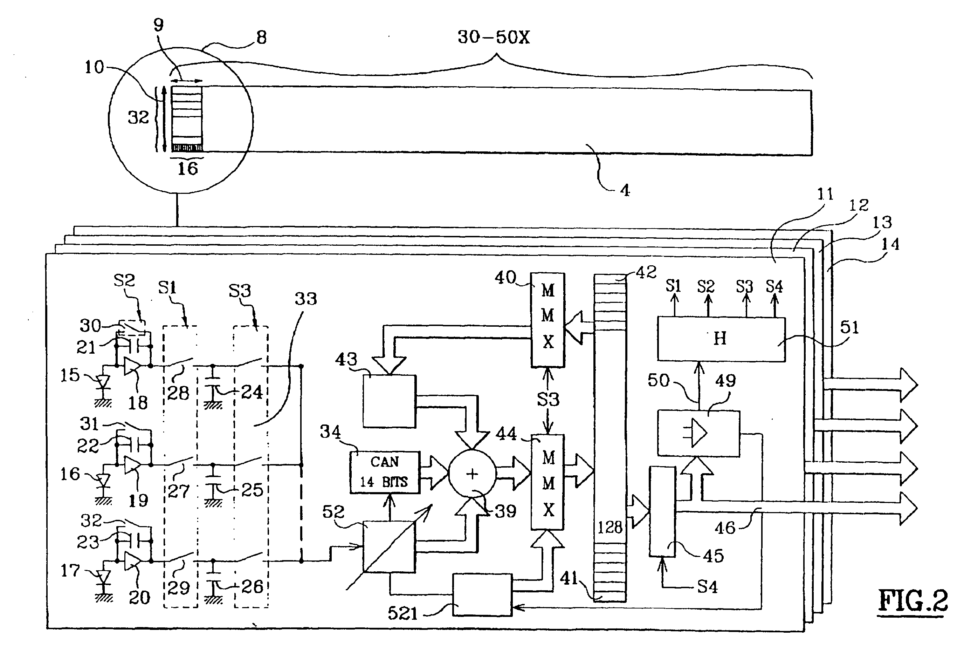

La figure 1 montre les éléments essentiels d'un tomodensitomètre. Ce tomodensitomètre est utilisable dans le cadre de l'invention. Ce tomodensitomètre comporte une source 1 produisant un rayonnement X 2 qui irradie un corps 3 interposé entre la source 1 et un détecteur 4. Le détecteur 4 peut comporter par ailleurs un détecteur auxiliaire 5 situé hors du champ X masqué par le corps 3. Le détecteur auxiliaire 5 peut servir à normaliser les mesures effectuées. Le tomodensitomètre tourne autour d'un axe de rotation dont on distingue la trace 6. Le détecteur 4 est un détecteur comportant une multiplicité d'éléments de détection 7.

Le détecteur 4 comporte une couche 71 d'éléments scintillateurs superposés à une couche 72 d'éléments de détection proprement dits. Les éléments scintillateurs de la couche 71 effectuent une conversion des rayons X en des rayons lumineux auxquels sont sensibles les éléments photodétecteurs de la couche sous-jacente de détection 72.

Selon la sensibilité admise actuellement, pour 1 photon X reçu dans la couche 71, environ 1000 photons lumineux sont produits par un élément de cristal scintillateur. Les éléments de cristal scintillateur sont séparés les uns des autres par des parois de transition d'un cristal à l'autre.

La figure 2 montre un exemple de réalisation des éléments de détection du détecteur 4. Ce détecteur 4 comporte un ensemble de modules 8. Les modules 8 sont des matrices d'éléments de détection. Les modules 8 sont placés côte à côte les uns les autres, dans le sens de la longueur du détecteur 4. Dans un exemple, la longueur 9 d'un module, est de l'ordre de 20 mm. Dans le même exemple, il peut y avoir entre 30 et 50 modules alignés dans le sens de la longueur du détecteur 4. Ceci amène à une longueur du détecteur comprise entre 60 cm et 1 mètre. Dans ce même exemple, la largeur 10 du module 8, dans sa partie utile à la détection, est de l'ordre de 64 mm. Ceci permet donc d'acquérir des coupes situées dans une épaisseur de l'ordre de 40 mm à l'intérieur du corps 3 interposé. Les exemples chiffrés qui sont donnés ci-après, n'ont pour but que de simplifier l'explication et ne peuvent pas conduire à une limitation de la portée de la protection obtenue par l'invention. Dans cet exemple, les modules comportent un arrangement de 32 lignes, empilées les unes sur les autres dans le sens de la largeur 10, et 16 colonnes mises côte à côte dans le sens de la longueur 9 du détecteur 4. De ce fait, le module 8 comporte 512 détecteurs élémentaires. Pour fixer les idées, on admettra par ailleurs que le tomodensitomètre tourne à une vitesse de deux tours par seconde et qu'on veut effectuer 1000 vues sur chaque tour. La durée d'une vue est donc de 500 microsecondes. Au cours de chacune des vues, au cours de chacune de ces 500 microsecondes, il convient de mesurer pour tous les modules, et pour tous les détecteurs élémentaires dans chaque module, le signal d'éclairement détecté.

Le bas de la figure 2 montre l'architecture de la réalisation des détecteurs d'un module 8. Chaque module de détecteurs est constitué de préférence de quatre sous-groupes 11 à 14 de détecteurs élémentaires et de circuits de traitements associés. Le sous-groupe 11 comporte ainsi 128 détecteurs notés 15 à 17. Dans cet exemple, ces détecteurs sont des diodes du type photovoltaïque. Les sous-groupes comportent par exemple des détecteurs situés dans huit colonnes adjacentes parmi seize et dans seize lignes parmi 32. En variante, on réalise deux sous-groupes par module.

Les photodiodes 15 à 17 sont des éléments de détection mis en place dans la couche 72 d'éléments photodétecteurs du détecteur 4. Elles reçoivent un rayonnement lumineux correspondant au rayonnement X reçu à l'endroit du cristal scintillateur qui les surmonte. Ces diodes sont reliées par leurs deux bornes, d'une part, en commun à la masse, et d'autre part, d'une manière individualisée, chacune à l'entrée d'un amplificateur respectivement 18 à 20. Les amplificateurs 18 à 20 sont des amplificateurs de type opérationnel. Dans un exemple, ils peuvent être constitués par un simple transistor. Les amplificateurs 18 à 20 sont montés en intégrateurs par l'intermédiaire de condensateurs respectivement 21 à 23 qui rebouclent leurs sorties sur leurs entrées. Les sorties des amplificateurs 18 à 20 sont en outre reliées à des condensateurs de stockage respectivement 24 à 26. La liaison des sorties des amplificateurs 18 à 20 aux condensateurs 24 à 26 est réalisée par l'intermédiaire d'interrupteurs 27 à 29 commandés par un signal S1.

Au moment où les interrupteurs 27 à 29 sont fermés, les amplificateurs 18 à 20 chargent, instantanément, les condensateurs 24 à 26. La charge injectée dans les condensateurs est dépendante de la tension à laquelle ont abouti les intégrateurs 18 à 20 à l'issue d'une durée d'intégration (correspondant à une vue, correspondant donc à 500 microsecondes en pratique).

Lorsque la tension disponible aux sorties des amplificateurs 18 à 20 a été transmise aux condensateurs 24 à 26, les amplificateurs 18 à 20 sont réinitialisés par un signal S2 appliqué sur un jeu d'interrupteurs respectivement 30 à 32 en dérivation sur les condensateurs 21 à 23. La réinitialisation est instantanée. A l'issue du signal S2, les diodes 15 à 17 recommencent à injecter du courant dans les amplificateurs 18 à 20.

Un multiplexeur 33 permet de relier, chacun à leur tour, les condensateurs de stockage 24 à 26 à un convertisseur analogique numérique 34, qui, dans un exemple préféré de l'invention, est un convertisseur analogique numérique sur 14 bits.

Dans l'état de la technique représentée par les figures 3a à 3c qui montrent les synchronisations des signaux S1 à S3, le signal délivré par le convertisseur 34 était un signal relatif à une vue. Il était également différencié pour chacun des détecteurs 15 à 17. La figure 3c montre notamment qu'un signal S3 comportant 8 x 16 = 128 impulsions permettait, au cours d'une vue, d'échantillonner, chacun à leur tour, et de numériser les signaux contenus dans les 128 condensateurs 24 à 26. Avec les quatre sous-groupes 11 à 14, on disposait ainsi pour chaque vue des 4 x 128 = 512 mesures correspondant à un module. La durée de la vue est ici la durée qui sépare les impulsions 35 et 36 du signal S1, ou 37 et 38 du signal S2 dans les figures 3a et 3b.

On remarquera que, dans le module comportant 512 détecteurs élémentaires, il y a 4 convertisseurs 34 par module 8. Il y en a un pour chacun des sous-groupes 11 à 14. Compte tenu du nombre de modules, 50, il y a 200 convertisseurs 34 dans le dispositif de l'invention. En plus ces convertisseurs sont simples.

Compte tenu de la dynamique importante du signal à mesurer, et du nombre également important des convertisseurs 34, il convenait, selon l'invention, de préconiser des convertisseurs 34 de taille plus petite (à 14 bits) plutôt que des convertisseurs selon l'état de la technique (à 20 bits). Dans l'invention, pour résoudre ce problème, on a décidé d'augmenter la fréquence avec laquelle les convertisseurs 34 échantillonnent puis numérisent les contenus des condensateurs 24 à 26.

En correspondance, on augmente de la même façon la fréquence de transfert des tensions de sortie des amplificateurs 18 à 20 aux condensateurs 24 à 26. Ceci est montré sur les figures 3d à 3f correspondant respectivement aux figures 3a à 3c. On constate que dans ces groupements de figures, la durée D d'une vue reste la même. Dans un exemple, elle est toujours de 500 microsecondes. Par contre, dans l'invention, l'intégration, l'échantillonnage et la quantification sont exécutés n fois pendant cette période. Dans un exemple préféré, n = 8. La signification du signal S3 de la figure 3f est que le convertisseur 34 délivre les 128 résultats, n fois plus fréquemment que dans le cadre de la figure 3c.

Dans l'invention, en outre, on additionne dans un additionneur 39, au fur et à mesure les résultats, délivrés par le convertisseur 34 pour un détecteur, avec des résultats correspondant au même détecteur délivrés par ce même convertisseur, mais à une quantification précédente. Dans ce but, un premier multiplexeur 40 évoluant lui aussi au rythme du signal S3, prélève dans une mémoire 41, à une adresse 42 (évoluant au rythme du signal S3), le résultat d'une quantification précédente qui y avait été stockée. Ce résultat est stocké dans une mémoire tampon 43. La mémoire tampon 43 est en relation avec l'additionneur 39. Le moment venu, l'additionneur 39 additionne en correspondance le contenu de la mémoire 43 avec le résultat délivré par le convertisseur 34. La sortie de l'additionneur 39 est reliée à un deuxième multiplexeur 44 dont le rôle est de mémoriser, à nouveau à l'adresse 42, le résultat de l'addition de l'ancien contenu de l'adresse 42 avec le résultat de quantification délivré par le convertisseur 34.

La figure 3g montre un signal supplémentaire : le signal S4. Le signal S4 est le signal synchrone du signal S1 ou du signal S2 de l'état de la technique, Figures 3a et 3b. Le signal S4 commande un troisième multiplexeur 45 qui extrait rapidement de la mémoire 41 les 128 données qui y étaient stockées. La mémoire 41 comporte des cellules mémoires de préférence de 20 bits. La lecture de la mémoire 41 par le multiplexeur 45 doit être rapide. En effet, elle doit se produire pendant la première des n quantifications réalisées par le convertisseur analogique numérique 34 au cours de la vue.

Au besoin, le multiplexeur 45 est combiné avec le multiplexeur 40, le signal n'étant disponible sur la sortie 46 qu'une fois tous les n fois.

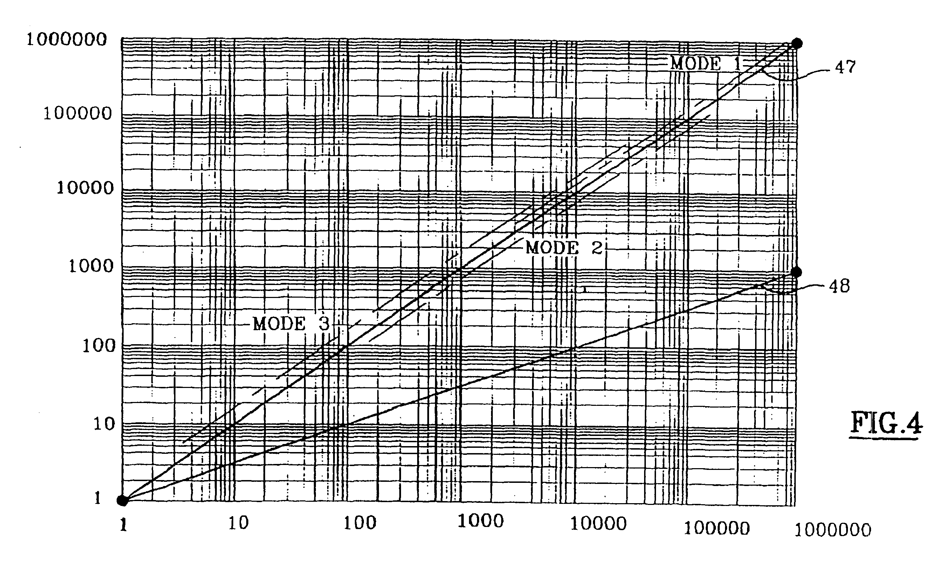

La figure 4 montre différents modes d'utilisation du tomodensitomètre de l'invention et de son système de détection. En abscisse, elle comporte le nombre de photons X incidents pendant la durée d'une vue (500 microsecondes) sur un détecteur élémentaire du détecteur 4. En ordonnée, elle montre le signal de sortie attendu, après numérisation. Les échelles des axes de coordonnées sont logarithmiques pour tenir compte d'une dynamique globale importante à 20 bits. La droite 47 montre le traitement (normal) effectué par la chaîne de détection avec un taux de conversion de un pour un. La droite 48 montre par ailleurs l'évolution du rapport signal sur bruit résultant de la détection quantique par les cristaux scintillateurs. Notamment pour l'illumination nominale, le rapport signal sur bruit vaut 1000 (racine carrée de 1 million).

On a dessiné sur le diagramme de la figure 4, trois domaines intitulés respectivement Mode 1, Mode 2 et Mode 3 et correspondant à des plages d'illuminations X différentes. Pour le Mode 1, le signal détecté correspond à des charges comprises entre 12,5 pC et 100 pC. On utilise, selon l'invention, pour des signaux correspondant à ce mode, une accélération du rythme de prélèvement des tensions de sorties des amplificateurs 18, 19 et 20 ainsi que la quantification correspondante par le convertisseur 34. En définitive, dans ce cas, les convertisseurs 34 travailleront au rythme du signal S3 de la figure 3f. Dans le cas où n = 8, le résultat stocké dans la cellule 42 de la mémoire 41 vaudra à l'issue de la vue, un signal codé sur 17 bits (14+3) placés dans les bits de poids forts. En effet l'addition de huit signaux codés sur 14 bits conduit à un résultat sur 17 bits. L'additionneur 39 est donc un additionneur sur 17 bits.

Selon le perfectionnement de l'invention, on mesure dans un comparateur 49 relié à la sortie 46 du multiplexeur 45 un signal correspondant à chaque détecteur élémentaire d'un sous-groupe 11 de détecteurs élémentaires. Si le signal d'au moins un de ces détecteurs élémentaires est supérieur, en l'équivalence, à 12,5 pC, on utilise pour tous les détecteurs élémentaires de ce sous-groupe, l'acquisition accélérée. Par contre, si le signal de tous les détecteurs d'un sous-groupe est inférieur à 12,5 pC, on décide de ne plus mettre en oeuvre le perfectionnement de l'invention. Dans ce but, le comparateur 49 délivre un signal 50 dont le rôle est de transformer le signal S1 visible sur la figure 3d, en un signal S1 visible sur la figure 3a. En pratique, les fréquences des signaux S1, S2 et S3 sont alors divisées par n. Une horloge 51, jouant un rôle de séquenceur, délivre donc, en fonction du signal 50, des signaux S1 S2 S3 accélérés ou non. Il est facile, avec un compteur cyclique animé par une horloge très rapide, de scruter un bit du signal délivré par ce compteur et de constituer les impulsions S1 à S3 avec l'état de ce bit. Pour une accélération par huit, il suffit de scruter un bit de poids plus faible, de se décaler de trois unités.

De préférence, on ne provoque plus l'accélération lorsque le signal détecté est faible et que le bruit de numérisation par le convertisseur analogique numérique serait supérieur au bruit de quantification des rayons X dû au cristal scintillateur.

On a constaté par ailleurs que le gradient de l'absorption varie peu dans l'image. En pratique, le gradient est inférieur à 50 ou 100. Ceci signifie que le signal détecté sur les détecteurs d'un module 8 n'est pas trop différent du signal détecté sur les détecteurs d'un module adjacent. Ceci a conduit à constituer selon l'invention les groupes de détecteurs.

En outre, compte tenu de la rotation lente du tomodensitomètre, les zones du corps vues par des détecteurs adjacents dans le module 8, au cours d'une vue, sont vues quasiment par les mêmes détecteurs du module 8 à la vue suivante. Ainsi on est capable de prédire sur une vue ce que va être le signal sur une vue suivante, car localement le signal va de plus peu varier, le contraste n'étant pas à évolution brutale. Ceci permet alors, à titre de perfectionnement, lorsqu'on fait une mesure pour une vue, de décider d'appliquer le signal 50, non pas au cours de la vue, mais pour une vue suivante. Autrement dit, le signal 50 provoquant le basculement du Mode 1 au Mode 2, ou réciproquement, n'est appliqué à l'horloge 51 qu'après l'impulsion du signal S4 qui marque la fin de la vue en cours.

Dans certains cas, le signal détecté est beaucoup plus faible qu'un équivalent à 1,6 pC. Dans ce cas, afin d'utiliser le convertisseur 34 au maximum de sa gamme, on amplifie préalablement avec un amplificateur 52, avant conversion, le signal en provenance des éléments de stockage 24 à 26. On effectue cette amplification de préférence si tous les signaux de détecteur d'un groupe sont inférieurs à un seuil. Par exemple, l'amplification sera dans un rapport 8. Autrement dit, avec une telle amplification on utilise totalement la dynamique du convertisseur 34. Cependant, du fait de l'amplification réalisée, il convient alors de décaler, dans les cellules 42 de la mémoire 41, le résultat vers les bits de poids faibles. Si le facteur d'amplification vaut 8, il faudra décaler le résultat de 3 bits vers les poids faibles. En réalité, le facteur d'amplification ne sera pas de huit. En conséquence on divisera le résultat de la conversion par le facteur d'amplification réel. Ceci sera réalisé par un circuit interposé entre le convertisseur 34 et l'additionneur 39.

On notera que les seuils de 12,5 pC et 1,6 pC sont arbitraires, quoique assez bien adaptés au problème.

Le signal issu du comparateur 49 qui commande l'amplificateur 52, est par ailleurs mémorisé dans un circuit de sélection 521. Le circuit 521 est alors en relation avec le multiplexeur 44 pour provoquer, au moment de l'enregistrement en mémoire 41, un enregistrement décalé ou non de 3 bits vers les bits de poids faible.

En définitive, on aura obtenu ainsi une dynamique de 14 + 3 + 3 = 20 bits en n'utilisant jamais qu'un convertisseur analogique numérique capable d'une dynamique de 14 bits.

Au besoin, l'amplification par l'amplificateur 52 et l'accélération du rythme peuvent être combinées.

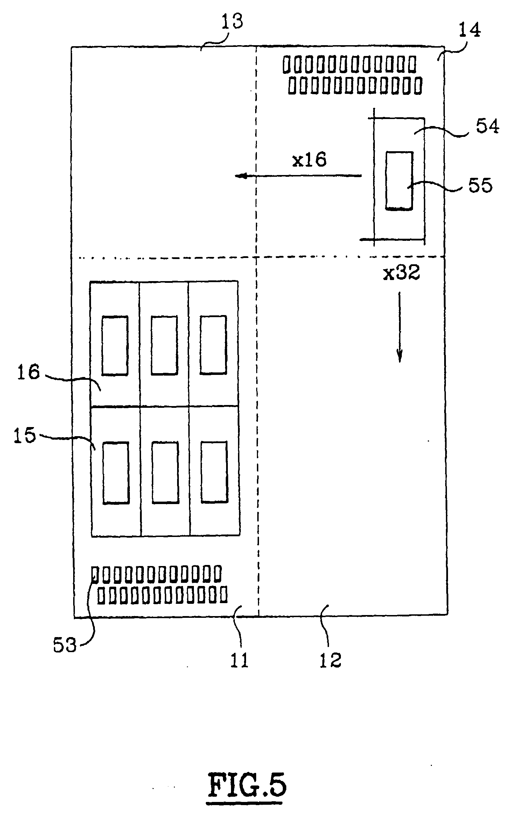

La figure 5 montre un exemple pratique de réalisation d'un module dans le cas où le détecteur comporterait des photodiodes de type photovoltaïques. La figure 5 montre un module 8 comportant 512 détecteurs élémentaires répartis en 32 lignes et 16 colonnes. La figure 5 montre les 4 sous-groupes 11, 12, 13 et 14 comportant chacun 128 photodétecteurs. Dans un exemple, ces photodétecteurs formant la couche 72 sont réalisés sur un substrat silicium porté par un support céramique qui va de plus porter les circuits de commande. Des métallisations comme 53 sont reliées par des connexions (non représentées) chacune à un photodétecteur individualisé. L'autre borne des ces photodétecteurs est reliée à la masse, une métallisation non représentée sous-jacente au substrat et commune à tous.

Dans un exemple, les photodétecteurs correspondent à une surface de 0,6 mm par 1,2 mm, inférieure aux dimensions de 1,25 mm par 2 mm données précédemment. Il y a donc un cadre neutralisé autour de chaque zone détectrice 55. Ce cadre neutralisé a pour but d'isoler électriquement les photodétecteurs entre eux et d'autre part de s'adapter à la taille des cristaux scintillateurs déposés sur un photodétecteur. Il n'y a pas véritablement d'inconvénient à agir de cette façon puisque le phénomène de détection quantique se produit dans un cristal scintillateur de la couche 71 qui surmonte le détecteur 55 et le cadre 54. La photodiode 15 est quant à elle sensible à un rayonnement lumineux comportant, approximativement, 1000 fois plus de photons. Dans ce cas, les surfaces utiles des diodes sont inférieures à la surface correspondante présentée par un cristal scintillateur qui les surmonte.

## Revendications

1. - Procédé d'acquisition de mesures dans un tomodensitomètre comportant une source (1) de rayonnement X destiné à irradier un corps (3) pendant une vue (D) donnée dans lequel :
- on mesure (24-26) pour cette vue, dans chaque détecteur d'un réseau (11) de détecteurs, un signal analogique représentant l'effet (15-17) de l'absorption du rayonnement X dans le corps à l'endroit de chacun de ces détecteurs,
- on échantillonne (33) et on convertit (34) chaque signal analogique de détecteur en un signal numérique de détecteur,
**caractérisé en ce que**
- on échantillonne et on convertit le signal analogique de chaque détecteur à n reprises au cours de la vue, et
- on additionne (39) les n signaux convertis de chaque détecteur pour constituer (S4) le signal numérique de vue de chaque détecteur.

2. - Procédé selon la revendication 1, **caractérisé en ce que**
- on constitue (11-14) des groupes de détecteurs,
- on compare (49) le signal d'au moins un détecteur d'un groupe à un seuil, et
- on échantillonne à n reprise les signaux des détecteurs du groupe si ce signal d'au moins un détecteur du groupe est supérieur à ce seuil.

3. - Procédé selon l'une des revendications 1 à 2, **caractérisé en ce que**
- on constitue (11-14) des groupes de détecteurs,
- on compare les signaux de tous les détecteurs d'un groupe à un seuil, et
- on amplifie (52) avant conversion les signaux des détecteurs du groupe si chacun de ces signaux est inférieur à un seuil.

4. - Procédé selon l'une des revendications 2 ou 3, **caractérisé en ce que**
- on échantillonne à n reprises ou on amplifie des signaux analogiques de détecteurs qui correspondent à une vue ultérieure à celle pendant laquelle on fait la comparaison.

5. - Procédé selon l'une des revendications 1 à 4, **caractérisé en ce que** les détecteurs sont des diodes photovoltaïques et **en ce que** pour mesurer
- on intègre (18-20) dans un intégrateur, à titre de signal de détecteur, un signal de courant produit par chaque diode photovoltaïque pendant une durée n fois plus faible que la durée d'une vue avant d'échantillonner (S1) ce signal de détecteur, et
- on remet à zéro (S2) l'intégrateur avant une nouvelle intégration de ce courant.

6. - Tomodensitomètre muni d'un dispositif d'acquisition de mesures comportant
- un tube (1) a rayons X pour irradier avec un rayonnement X (2) un corps (3) pendant des vues (D) successives,
- un réseau (4) de détecteurs (7) pour mesurer un signal analogique représentant l'effet de l'absorption du rayonnement X dans le corps à l'endroit de chacun de ces détecteurs pendant une vue,
- un convertisseur (34) analogique numérique pour échantillonner et convertir chaque signal analogique de détecteur en un signal numérique de détecteur,
- un séquenceur (51) pour piloter le réseau de détecteurs et le convertisseur au rythme des vues,
**caractérisé en ce qu'**il comporte
- des moyens (50) dans le séquenceur pour piloter le réseau de détecteurs et le convertisseur à un rythme n fois plus élevé que le rythme des vues, et
- un additionneur (39) pour additionner les n signaux convertis de chaque détecteur pour constituer (S4) le signal numérique de vue de chaque détecteur.

7. - Tomodensitomètre selon la revendication 6, **caractérisé en ce qu'**il comporte
- des groupes (11-14) de détecteurs,
- un comparateur (49) affecté à un groupe pour comparer un signal d'au moins un détecteur d'un groupe à un seuil, et
- un circuit (50-51) pour commander le pilotage à un rythme n fois plus élevé si ce signal d'au moins un détecteur du groupe est supérieur à ce seuil.

8. - Tomodensitomètre selon l'une des revendications 6 ou 7, **caractérisé en ce qu'**il comporte
- des groupes (11-14) de détecteurs,
- un comparateur (49) affecté à un groupe pour comparer les signaux de tous les détecteurs d'un groupe à un seuil, et
- un amplificateur (52) pour amplifier avant conversion les signaux des détecteurs du groupe si ces signaux sont inférieurs à un seuil.

9. - Tomodensitomètre selon l'une des revendications 7 à 8, **caractérisé en ce qu'**un groupe (8) de détecteurs comporte deux ou quatre sous-groupes (11-14) de détecteur montés avec leurs circuits de commande tous ensemble sur un même support, par exemple un support céramique.

10. - Tomodensitomètre selon l'une des revendications 6 à 9, **caractérisé en ce que**
- les détecteurs sont des diodes photovoltaïques associées chacune à un intégrateur (18) commandé (S1, S2).

11. - Tomodensitomètre selon l'une des revendications 6 à 10, **caractérisé en ce que** les détecteurs comportent des photodiodes dont la surface de détection est inférieure à la surface de détection d'un cristal scintillateur qui les surmonte.

## Claims

1. Process for acquiring measurements with a tomodensitometer which includes a source (1) of X-rays intended to irradiate a body (3), during a given view (D) in which:
- an analogue signal is measured (24-26) for this view, in each detector of an array (11) of detectors, this signal representing the effect (15-17) of the absorption of the X-ray radiation in the body at the location of each of these detectors,
- each analogue detector signal is sampled (33) and converted (34) into a digital detector signal,
**characterized in that**
- the analogue signal from each detector is sampled with n repetitions in the course of the view, and
- the n converted signals from each detector are added together (39) to construct (S4) the digital view signal of each detector.

2. Process according to Claim 1, **characterized in that**
- groups of detectors are constructed (11-14),
- the signal from at least one detector of a group is compared (49) with a threshold, and
- the signals from the detectors of the group are sampled with n repetitions if this signal from at least one detector of the group is above this threshold.

3. Process according to one of Claims 1 to 2, **characterized in that**
- groups of detectors are constructed (11-14),
- the signals from all the detectors of a group are compared with a threshold, and
- the signals from the detectors of the group are amplified (52) before conversion if each of these signals is below a threshold.

4. Process according to one of Claims 2 and 3, **characterized in that**
- analogue signals from detectors which correspond to a view subsequent to that during which the comparison is made are sampled with n repetitions or are amplified.

5. Process according to one of Claims 1 to 4, **characterized in that** the detectors are photovoltaic diodes and **in that** in order to make measurements
- there is integrated (18-20) in an integrator, in the guise of detector signal, a current signal produced by each photovoltaic diode over a duration n times smaller than the duration of a view before sampling (S1) this detector signal, and
- the integrator is reset to zero (S2) before a new integration of this current.

6. Tomodensitometer furnished with a device for acquiring measurements comprising
- an X-ray tube (1) for irradiating a body (3) with X-ray radiation (2) during successive views (D),
- an array (4) of detectors (7) for measuring an analogue signal representing the effect of the absorption of the X-ray radiation in the body at the location of each of these detectors during a view,
- an analogue/digital converter (34) for sampling and converting each analogue detector signal into a digital detector signal,
- a sequencer (51) for driving the array of detectors and the converter at the rate of the views,
**characterized in that** it includes
- means (50) in the sequencer for driving the array of detectors and the converter at a rate n times greater than the rate of the views and,
- an adder (39) for adding together the n converted signals from each detector so as to construct (S4) the digital view signal of each detector.

7. Tomodensitometer according to Claim 6, **characterized in that** it includes
- groups (11-14) of detectors,
- a comparator (49) assigned to a group so as to compare a signal from at least one detector of a group with a threshold, and
- a circuit (50-51) for controlling the driving at a rate n times greater if this signal from at least one detector of the group is above this threshold.

8. Tomodensitometer according to one of Claims 6 and 7, **characterized in that** it includes
- groups (11-14) of detectors,
- a comparator (49) assigned to a group for comparing the signals from all the detectors of a group with a threshold, and
- an amplifier (52) for amplifying before conversion the signals from the detectors of the group of these signals are below a threshold.

9. Tomodensitometer according to one of Claims 7 to 8, **characterized in that** a group (8) of detectors includes two or four detector subgroups (11-14) mounted with their control circuits all together on the same support, for example a ceramic support.

10. Tomodensitometer according to one of Claims 6 to 9, **characterized in that**
- the detectors are photovoltaic diodes each associated with a controlled (S1, S2) integrator (18).

11. Tomodensitometer according to one of Claims 6 to 10, **characterized in that** the detectors include photo diodes whose detection area is less than the detection area of a scintillator crystal which surmounts them.

## Patentansprüche

1. Verfahren zur Erfassung von Messungen in einem Computertomographen mit einer Quelle (1) einer Röntgenstrahlung, die dazu bestimmt ist, einen Körper (3) während einer gegebenen Aufnahme (D) zu bestrahlen, bei dem:
- für diese Aufnahme in jedem Detektor eines Netzes (11) von Detektoren ein analoges Signal gemessen wird (24-26), das die Wirkung (15-17} der Absorption der Röntgenstrahlung im Körper an der Stelle jedes der Detektoren darstellt,
- jedes analoge Detektorsignal getastet (33) und in ein digitales Detektorsignal umgewandelt wird (34),
**dadurch gekennzeichnet, dass**
- das analoge Signal jedes Detektors während der Aufnahme n Mal getastet und umgewandelt wird, und
- die n umgewandelten Signale jedes Detektors addiert werden (39), um das digitale Aufnahmesignal jedes Detektors zu bilden (S4).

2. Verfahren nach Anspruch 1, **dadurch gekennzeichnet, dass**
- Gruppen von Detektoren gebildet werden (11-14),
- das Signal mindestens eines Detektors einer Gruppe mit einem Schwellwert verglichen wird (49), und
- die Signale der Detektoren der Gruppe n Mal getastet werden, wenn dieses Signal mindestens eines Detektors der Gruppe höher ist als dieser Schwellwert.

3. Verfahren nach einem der Ansprüche 1 bis 2, **dadurch gekennzeichnet, dass**
- Gruppen von Detektoren gebildet werden (11-14),
- die Signale aller Detektoren einer Gruppe mit einem Schwellwert verglichen werden, und
- vor der Umwandlung die Signale der Detektoren der Gruppe verstärkt werden (52), wenn jedes dieser Signale unter einem Schwellwert liegt.

4. Verfahren nach einem der Ansprüche 2 oder 3, **dadurch gekennzeichnet, dass**
- analoge Signale von Detektoren, die einer späteren Aufnahme als derjenigen, während der der Vergleich durchgeführt wird, entsprechen, n Mal getastet oder verstärkt werden.

5. Verfahren nach einem der Ansprüche 1 bis 4, **dadurch gekennzeichnet, dass** die Detektoren fotovoltaische Dioden sind, und dass zur Messung
- in eine Integrierschaltung als Detektorsignal ein Stromsignal integriert wird (18-20), das von jeder fotovoltaischen Diode während einer Dauer erzeugt wird, die n Mal kürzer ist als die Dauer einer Aufnahme, ehe dieses Detektorsignal getastet wird (S1), und
- die Integrierschaltung vor einer neuen Integration dieses Stroms auf Null zurückgesetzt wird (S2).

6. Computertomograph, der mit einer Vorrichtung zur Erfassung von Messungen ausgestattet ist und aufweist
- eine Röntgenstrahlröhre (1), um einen Körper (3) während aufeinander folgender Aufnahmen (D) mit einer Röntgenstrahlung (2) zu bestrahlen,
- ein Netz (4) von Detektoren (7), um ein analoges Signal zu messen, das die Wirkung der Absorption der Röntgenstrahlung im Körper an der Stelle jedes der Detektoren während einer Aufnahme darstellt,
- einen Analog/Digital-Wandler (34), um jedes analoge Detektorsignal zu tasten und in ein digitales Detektorsignal umzuwandeln,
- eine Ablaufsteuerung (51), um das Netz von Detektoren und den Wandler im Rhythmus der Aufnahmen zu steuern,
**dadurch gekennzeichnet, dass** er aufweist
- Mittel (50) in der Ablaufsteuerung, um das Netz von Detektoren und den Wandler mit einem Rhythmus zu steuern, der n Mal schneller ist als der Rhythmus der Aufnahmen, und
- ein Addierglied (39), um die n umgewandelten Signale jedes Detektors zu addieren, um das digitale Aufnahmesignal jedes Detektors zu bilden (S4).

7. Computertomograph nach Anspruch 6, **dadurch gekennzeichnet, dass** er aufweist
- Gruppen (11-14) von Detektoren,
- einen Vergleicher (49), der einer Gruppe zugeteilt ist, um ein Signal mindestens eines Detektors einer Gruppe mit einem Schwellwert zu vergleichen, und
- eine Schaltung (50-51), um die Steuerung mit einem n mal höheren Rhythmus zu betätigen, wenn dieses Signal mindestens eines Detektors der Gruppe höher ist als dieser Schwellwert.

8. Computertomograph nach einem der Ansprüche 6 oder 7, **dadurch gekennzeichnet, dass** er aufweist
- Gruppen (11-14) von Detektoren,
- einen Vergleicher (49), der einer Gruppe zugeteilt ist, um die Signale aller Detektoren einer Gruppe mit einem Schwellwert zu vergleichen, und
- einen Verstärker (52), um vor der Umwandlung die Signale der Detektoren der Gruppe zu verstärken, wenn diese Signale unter einem Schwellwert liegen.

9. Computertomograph nach einem der Ansprüche 7 bis 8, **dadurch gekennzeichnet, dass** eine Gruppe (8) von Detektoren zwei oder vier Untergruppen (11-14) von Detektoren aufweist, die mit ihren Steuerschaltungen alle zusammen auf den gleichen Träger montiert sind, zum Beispiel einen keramischen Träger.

10. Computertomograph nach einem der Ansprüche 6 bis 9, **dadurch gekennzeichnet, dass**
- die Detektoren fotovoltaische Dioden sind, die je einer gesteuerten (S1 bis S2) Integrierschaltung (18) zugeordnet sind.

11. Computertomograph nach einem der Ansprüche 6 bis 10, **dadurch gekennzeichnet, dass** die Detektoren Fotodioden aufweisen, deren Erfassungsfläche kleiner ist als die Erfassungsfläche eines Szintillatorkristalls, der über ihnen angeordnet ist.
